(19) Europäisches Patentamt
European Patent Office
Office européen des brevets

(11) Numéro de publication : **0 169 186**
**B1**

(12) # FASCICULE DE BREVET EUROPÉEN

(45) Date de publication du fascicule du brevet : **23.03.88**

(51) Int. Cl.[4] : **C 07 D215/22**, C 07 D405/12, A 61 K 31/47

(21) Numéro de dépôt : **85870083.4**

(22) Date de dépôt : **11.06.85**

(54) **Nouveaux dérivés de décahydroquinoléinol, leur procédé de préparation et les compositions les contenant.**

(30) Priorité : **13.06.84 FR 8409242**

(43) Date de publication de la demande :
**22.01.86 Bulletin 86/04**

(45) Mention de la délivrance du brevet :
**23.03.88 Bulletin 88/12**

(84) Etats contractants désignés :
**AT CH DE FR GB IT LI LU NL SE**

(56) Documents cités :
**EP-A- 0 026 168**
**FR-A- 2 160 939**
**Le dossier contient des informations techniques présentées postérieurement au dépôt de la demande et ne figurant pas dans le présent fascicule.**

(73) Titulaire : **SANOFI, société anonyme**
**40, Avenue George V**
**F-75008 Paris (FR)**

(72) Inventeur : **Prost, Maurice**
**Avenue des Camélias, 48**
**B-1150 Bruxelles (BE)**
Inventeur : **de Claviere, Michel**
**2, rue de Fontardies**
**F-34680 St. Georges d'Orques (FR)**
Inventeur : **Polster, Peter**
**Rue Champ d'Oiseaux, 9bis**
**B-5990 Hamme-Mille (BE)**

(74) Mandataire : **Polus, Camille**
**c/o Cabinet Lavoix 2, Place d'Estienne d'Orves**
**F-75441 Paris Cedex 09 (FR)**

**EP 0 169 186 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention se rapporte, d'une manière générale, à de nouveaux composés hétérocycliques et, en particulier, à de nouveaux dérivés de décahydroquinoléinol ainsi qu'à leur procédé de préparation.

Les dérivés de décahydroquinoléinol de l'invention peuvent être représentés par la formule générale :

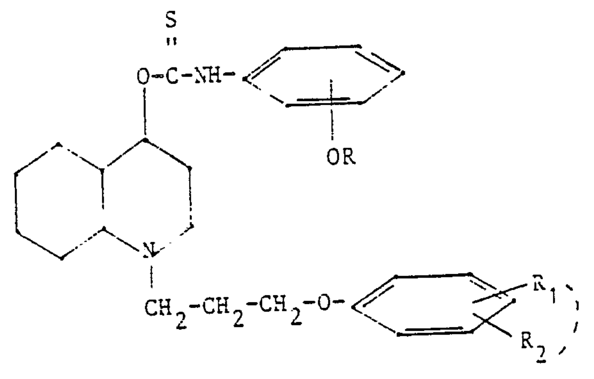

(I)

dans laquelle R représente un radical alkyle ayant de 1 à 3 atomes de carbone, $R_1$ représente fluor, chlore, brome ou méthoxy, $R_2$ représente hydrogène ou méthoxy ou $R_1$ et $R_2$, lorsqu'ils sont pris ensemble, représentent un groupement méthylènedioxy-2,3 ou méthylènedioxy-3,4.

L'invention se rapporte également aux sels d'addition non toxiques des dérivés de formule I, par exemple le chlorhydrate.

Les composés de formule I possèdent en position 4 du cycle décahydroquinoléine un radical thiocarbamoyloxy qui peut présenter une configuration axiale ou équatoriale.

L'invention se rapporte à la fois aux épimères axiaux et équatoriaux en question pris individuellement ou sous forme de mélanges.

Les dérivés de décahydroquinoléinol de l'invention possèdent de remarquables propriétés pharmacologiques notamment des propriétés inhibitrices de la translocation calcique, susceptibles de les rendre particulièrement utiles dans le traitement de certains syndromes pathologiques du système cardiovasculaire. Un autre objet de l'invention se rapporte, en conséquence, à des compositions pharmaceutiques ou vétérinaires contenant comme principe actif, au moins un dérivé de décahydroquinoléinol de l'invention, en association avec un véhicule pharmaceutique ou un excipient approprié.

Selon la voie d'administration choisie, la posologie journalière pour un être humain pesant 60 kg, se situera entre 2 et 500 mg de principe actif selon l'invention.

Les composés de formule I peuvent être préparés à partir d'hydroxy-4 transdécahydroquinoléines N-substituées de formule générale :

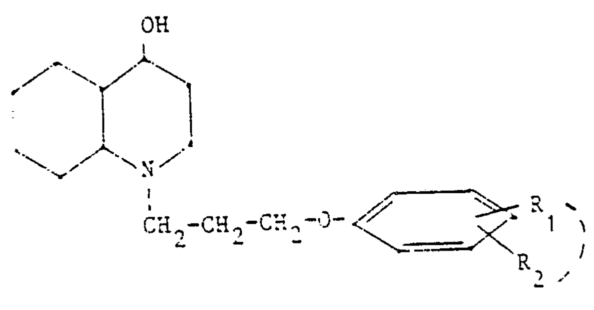

(II)

dans laquelle $R_1$ et $R_2$ ont la même signification que précédemment, par réaction, à la température ambiante et dans un solvant organique inerte tel que par exemple le benzène, le toluène, le chlorure de méthylène ou le chloroforme, avec un composé de formule générale :

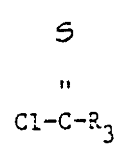

(III)

dans laquelle $R_3$ représente un atome de chlore ou un radical phénoxy pour obtenir un dérivé ou thiocarbonyloxy de formule générale :

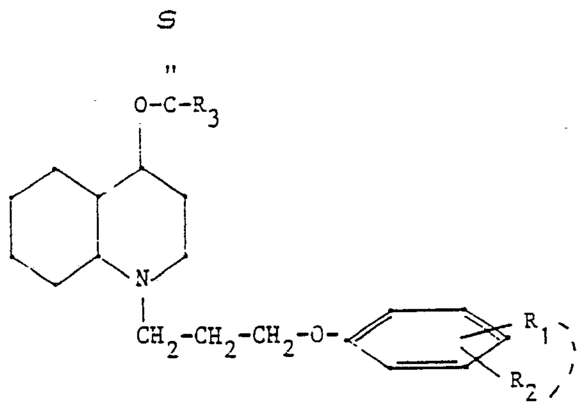

(IV)

dans laquelle $R_1$, $R_2$ et $R_3$ ont la même signification que précédemment, que l'on condense avec une amine primaire de formule générale :

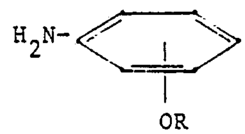

(V)

dans laquelle R a la même signification que précédemment, dans un solvant inerte et à la température ambiante, pour former l'ester thiocarbamique de formule I sous forme de sa base libre.

Lorsque $R_3$ représente un radical phénoxy, la réaction pour l'obtention du composé de formule IV est effectuée en présence d'une amine tertiaire comme par exemple la pyridine tandis que la condensation des composés de formule IV et V a lieu dans un alcool comme solvant, par exemple dans le méthanol.

Lorsque $R_3$ représente un atome de chlore la condensation des composés de formules IV et V s'effectue dans un solvant tel que par exemple le toluène, le chlorure de méthylène ou le chloroforme.

Les sels d'addition non toxiques des composés de formule I peuvent être préparés, de manière classique, en faisant réagir le composé de formule I correspondant sous forme de sa base libre avec un acide organique ou inorganique approprié.

Les hydroxy-4 trans-décahydroquinoléines N-substituées de formule II peuvent être préparées par la méthode décrite par M. PROST et coll. dans Eur. J. Med. Chem. 1981, 16, 119, ou par d'autres procédés connus, par exemple à partir d'hydroxy-4 trans-décahydroquinoléine et d'un composé halogéné de formule générale :

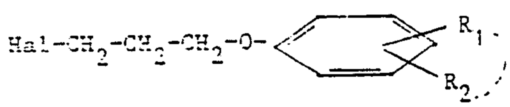

dans laquelle Hal représente un atome de chlore, de brome ou d'iode et $R_1$ et $R_2$ ont la même signification que précédemment.

Quant à l'hydroxy-4 trans-décahydroquinoléine de départ, il s'agit d'un produit également connu pouvant être préparé en réduisant l'oxo-4 trans-décahydroquinoléine suivant la méthode décrite par exemple dans Bull. Acad. Sci. U.S.S.R. 1962, 1599.

Cette méthode conduit à un mélange d'épimères axial et équatorial au niveau de l'hydroxyle. Ces isomères, sous forme séparée, ont été décrits par M. PROST et coll. dans Eur. J. Med. Chem. 1976, 11, (4), pp. 337-342.

En conséquence, les procédés décrits ci-dessus pour la préparation des dérivés de formule I au départ d'hydroxy-4 trans-décahydroquinoléine sont applicables soit à l'épimère axial soit à l'épimère équatorial de l'hydroxy-4 trans-décahydroquinoléine pour la préparation des épimères correspondants de formule I.

De même, les procédés en question sont applicables au mélange d'épimères axial et équatorial d'hydroxy-4 trans-décahydroquinoléine, obtenu par exemple après réduction de l'oxo-4 trans-décahydroquinoléine, en vue de préparer les composés de formule I sous forme d'un mélange d'épimères axial et équatorial.

On a décrit dans le brevet européen No. 0 026 168 des dérivés antiarythmiques de carbamoyloxy-4 (phénoxy-3 propyl)-1 trans-décahydroquinoléine substitués au niveau du groupement carbamoyloxy notamment par un radical bromo-4 phényle.

De même, on a cité dans le brevet français No. 2.160.939 des dérivés de [(fluoro-4 phényl)-4 oxo-

butyl]-1 trans-décahydroquinoléine comportant en position 1 un groupement carbamoyloxy substitué ou thiocarbamoyloxy substitué, ces dérivés présentant notamment des propriétés antiadrénergiques.

On a trouvé, dans le cadre de la présente invention, que des composés de structure analogue aux composés cités dans le brevet européen No. 0 026 168 mais dans lesquels le radical bromo-4 phényle est remplacé par un radical alkoxy phényle en particulier un radical méthoxy-4 phényle possèdent des propriétés inhibitrices de la translocation calcique au niveau membranaire de loin supérieures à celles mises en évidence chez les dérivés carbamoyloxy de l'état de la technique.

Dans certains cas, les composés de l'invention se sont révélés plus de dix fois plus actifs qu'un des composés connus à savoir la [(bromo-4 phényl) carbamoyloxy]-4 [(fluoro-4 phénoxy)-3 propyl]-trans-décahydroquinoléine (forme axiale) décrite dans le brevet européen No. 0 026 168.

Les composés de l'invention se sont également montré beaucoup plus actifs que le [(méthyl-4 phényl) carbamoyloxy]-4 [(fluoro-4 phénoxy)-3 propyl]-1 trans-décahydroquinoléine (forme axiale), composé couvert par le brevet européen No. 0 026 168 mais non spécifiquement cité.

Ces propriétés sont capables de rendre les composés en question très utiles dans le traitement de certains syndromes pathologiques du système cardiovasculaire, en particulier dans le traitement de l'angine de poitrine, de l'arythmie cardiaque ou encore dans le traitement de l'hypertension.

En outre, la toxicité présentée par les composés de l'invention s'est montrée compatible avec leur utilisation à des fins thérapeutiques.

Les propriétés inhibitrices calciques des composés de l'invention ont été mises en évidence par mesure de leur action antagoniste vis-à-vis de la réponse contractile à la dépolarisation provoquée par le potassium sur l'aorte isolée de rat (M. SPEDDING, Naunyn-Schmiedeberg's Arch. Pharmakol. 1982, 318, 234).

Il est bien établi que la dépolarisation de la membrane d'un muscle lisse par le potassium rend cette dernière perméable au calcium extracellulaire et provoque la contraction musculaire.

Dès lors, la mesure de l'inhibition de la réponse contractile à la dépolarisation par le potassium ou la mesure d'un relâchement de la contraction tonique à la dépolarisation potassique peut constituer une évaluation de la puissance d'un composé en tant qu'inhibiteur de la perméabilité membranaire aux ions $Ca^{++}$.

La technique utilisée est la suivante :

Sur des rats mâles Wistar pesant environ 425 g, on prélève l'aorte et on la coupe en bandelettes d'environ 40 mm de longueur et 3 mm de largeur. On place ces fragments dans une cuve à organe isolé de 25 ml contenant une solution de Krebs-bicarbonate modifiée (NaCl 112 mM ; KCl 5 mM ; $NaHCO_3$ 25 mM ; $KH_2PO_4$ 1 mM ; $MgSO_4$ 1,2 mM ; $CaCl_2$ 2,5 mM ; glucose 11,5 mM ; eau distillée jusqu'à 1 000 ml), parcourue par un courant de carbogène et maintenue à 37 °C. On relie la préparation à un microcapteur de force et on enregistre la réponse contractile après amplification. On applique une tension de 2 g à la préparation. On maintient la préparation durant 60 minutes dans la solution de Krebs-bicarbonate modifiée puis on provoque des contractions en remplaçant la solution de Krebs-bicarbonate par une solution de Krebs-potassique (NaCl 17 mM ; KCl 100 mM ; $NaHCO_3$ 25 mM ; $KH_2PO_4$ 1 mM ; $MgSO_4$ 1,2 mM ; $CaCl_2$ 2,5 mM ; glucose 11,5 mM ; eau distillée jusqu'à 1 000 ml). Lorsque la réponse contractile de la préparation est devenue reproductible, on introduit, dans le bain, une quantité égale à $10^{-7}$ ou $10^{-8}$ mole d'un composé de l'invention. Soixante minutes plus tard, un nouveau spasme est provoqué par la dépolarisation potassique.

Les résultats obtenus sur l'aorte éprouvée sont alors exprimés en % de l'effet contracturant maximal avant l'incubation avec la substance à tester. A titre d'exemples, les résultats suivants ont été obtenus, les composés de formule I étant sous forme axiale et sous forme de chlorhydrate.

| R | $R_1$ | $R_2$ | % de l'effet contracturant maximal $10^{-7}$ M | $10^{-8}$ M |
|---|---|---|---|---|
| $CH_3$ | F | H | 25,6 | 85,8 |
| $CH_3$ | Br | H | 49,2 | |
| $CH_3$ | $OCH_3$ | H | 6,8 | 44,2 |
| $CH_3$ | $OCH_3$ | $OCH_3$ | 28,7 | 82,6 |
| $CH_3$ | $O-CH_2-O$ | | 9,8 | 75,3 |

Un essai comparatif effectué, dans les mêmes conditions avec le chlorhydrate de [(méthyl-4 phényl) carbamoyloxy]-4[(fluoro-4 phénoxy)-3 propyl]-1 trans-décahydroquinoléine (forme axiale) a montré un

effet contracturant maximal de 74,3 %, à la dose de $10^{-7}$ mole.

Les compositions thérapeutiques de l'invention peuvent être présentées sous toute forme convenant à l'administration en thérapie humaine ou vétérinaire. Pour ce qui concerne l'unité d'administration, celle-ci peut prendre la forme, par exemple, d'un comprimé, d'une dragée, d'une capsule, d'une gélule, d'une poudre, d'une suspension ou d'un sirop pour l'administration orale, d'un suppositoire pour l'administration rectale ou d'une solution ou suspension pour l'administration parentérale.

Les compositions thérapeutiques de l'invention pourront comprendre, par unité d'administration, par exemple de 15 % à 50 % en poids d'ingrédient actif pour l'administration orale, de 3 % à 15 % d'ingrédient actif pour l'administration rectale et de 3 % à 5 % d'ingrédient actif pour l'administration parentérale.

Suivant la voie d'administration choisie, les compositions pharmaceutiques ou vétérinaires de l'invention seront préparées en associant au moins un des composés de l'invention avec un excipient approprié, ce dernier pouvant être constitué par exemple d'au moins un ingrédient sélectionné parmi les substances suivantes :

lactose, amidons, talc, stéarate de magnésium, polyvinylpyrrolidone, acide alginique, silice colloïdale, eau distillée, alcool benzylique ou agents édulcorants.

Les Exemples, non limitatifs suivants, illustrent l'invention :

Exemple 1

Chlorhydrate de [(fluoro-4 phénoxy)-3 propyl]-1[N-(méthoxy-4 phényl) thiocarbamoyloxy]-4 trans-décahydroquinoléine (forme axiale).

On prépare une solution de 40 g de [(fluoro-4 phénoxy)-3 propyl]-1 hydroxy-4 trans-décahydroquinoléine (forme axiale) dans 300 ml de chlorure de méthylène et on introduit ensuite, entre 0 et 5 °C, une solution de 50 g de thiophosgène dans 100 ml de chlorure de méthylène.

L'addition terminée, on maintient le mélange réactionnel sous agitation à une température de 20 à 22 °C pendant 3 jours. On élimine l'excès de thiophosgène en soumettant le mélange réactionnel au vide d'une trompe à eau tout en maintenant une température de 20 à 25 °C par un léger chauffage. On réajuste le volume avec du chlorure de méthylène, on refroidit le mélange réactionnel entre 0 et 10 °C et on ajoute une solution de 60 g de méthoxy-4 aniline dans 400 ml de chlorure de méthylène.

L'addition terminée, on maintient le mélange réactionnel pendant 3 jours sous agitation à une température de 20 à 22 °C puis on ajoute de l'eau. Après décantation, on lave la phase organique avec 8 fois 1 l d'acide chlorhydrique 1N. Après séchage et décoloration au charbon actif, on élimine le solvant et on laisse cristalliser dans 150 ml d'acétone.

De cette manière, on obtient 24,3 g de chlorhydrate de [(fluoro-4 phénoxy)-3 propyl]-1 [N-(méthoxy-4 phényl) thiocarbamoyloxy]-4 trans-décahydroquinoléine (forme axiale).

Rendement : 36,8 %

P.F. : 220 ± 1 °C.

A partir des produits appropriés et en utilisant le procédé décrit ci-dessus, on a préparé les composés suivants :

Chlorhydrate de [(bromo-4 phénoxy)-3 propyl]-1 [N-(méthoxy-4 phényl) thiocarbamoyloxy]-4 trans-décahydroquinoléine (forme axiale)

P.F. : 149 ± 1 °C (acétate d'éthyle)

Chlorhydrate de [(méthoxy-4 phénoxy)-3 propyl]-1 [N-(méthoxy-4 phényl) thiocarbamoyloxy]-4 trans-décahydroquinoléine (forme axiale)

P.F. : 199 ± 1 °C (acétate d'éthyle)

Chlorhydrate de [(diméthoxy-3,4 phénoxy)-3 propyl]-1 [N-(méthoxy-4 phényl) thiocarbamoyloxy]-4 trans-décahydroquinoléine (forme axiale)

P.F. : 188 ± 1 °C (acétate d'éthyle)

Chlorhydrate de [N-(méthoxy-4 phényl) thiocarbamoyloxy]-4[(méthylènedioxy-3,4 phénoxy)-3 propyl]-1 trans-décahydroquinoléine (forme axiale)

P.F. : 204 ± 1 °C (acétate d'éthyle)

Exemple 2

Chlorhydrate de [(méthoxy-4 phénoxy)-3 propyl]-1[N-(méthoxy-4 phényl) thiocarbamoyloxy]-4 trans-décahydroquinoléine (forme axiale)

A une solution de 7,5 g d'hydroxy-4[(méthoxy-4 phénoxy)-3 propyl]-1 transdécahydroquinoléine (forme axiale) dans 50 ml de toluène et contenant 4,5 ml de pyridine, on ajoute à une température de 5 à 10 °C, une solution de 7 g de chlorure de phénoxythiocarbonyle dans 30 ml de toluène.

L'addition terminée, on maintient le mélange réactionnel sous agitation à une température de 20 à 22 °C pendant 3 jours, puis on coule le mélange réactionnel sur 200 ml d'une solution saturée de bicarbonate de sodium. On décante et on réextrait la phase aqueuse avec un mélange toluène/éther éthylique. On réunit les phases organiques et on les lave à l'eau. Après séchage, on évapore à siccité.

On reprend le résidu avec 50 ml de méthanol et, à 0 à 5 °C, on ajoute cette solution, à une solution saturée de méthoxy-4 aniline dans le méthanol. L'addition terminée, on maintient le mélange réactionnel sous agitation à une température de 20 à 22 °C pendant 3 jours. On chauffe ensuite à 50-60 °C. On évapore à siccité et on reprend le résidu avec de l'isopropanol. Par addition d'une solution d'acide chlorhydrique dans l'isopropanol, on forme alors le chlorhydrate. Après évaporation à siccité, on cristallise dans un mélange hexane/isopropanol.

De cette manière, on obtient le chlorhydrate de [(méthoxy-4 phénoxy)-3 propyl]-1[N-(méthoxy-4 phényl) thiocarbamoyloxy]-4 trans-décahydroquinoléine (forme axiale).

Rendement : 24 %

P.F. : 199 ± 1 °C (acétate d'éthyle).

Exemple 3

Suivant des techniques pharmaceutiques connues, on a préparé une gélule par association des ingrédients suivants :

| Ingrédient | mg |
|---|---|
| Composé de l'invention | 100 |
| Amidon de maïs | 384 |
| Talc | 10 |
| Silice colloïdale | 6 |
| | 500 |

**Revendications**

1. Dérivés de décahydroquinoléinol correspondant à la formule générale :

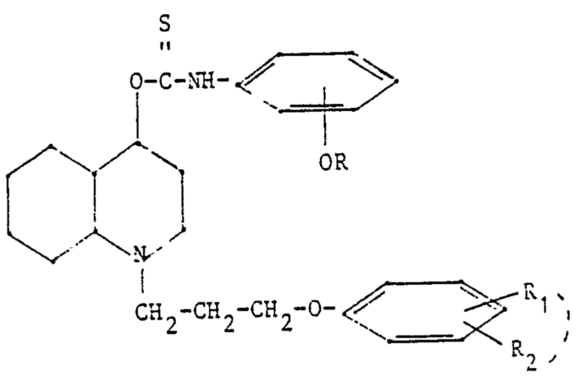

ainsi que leurs sels d'addition non toxiques, dans laquelle R représente un radical alkyle ayant de 1 à 3 atomes de carbone, $R_1$ représente fluor, chlore, brome ou méthoxy, $R_2$ représente hydrogène ou méthoxy ou $R_1$ et $R_2$, lorsqu'ils sont pris ensemble, représentent un groupement méthylènedioxy-2,3 ou méthylènedioxy-3,4.

2. Dérivés de décahydroquinoléinol selon la Revendication 1 sous forme d'épimères axiaux.

3. Dérivés de décahydroquinoléinol selon la Revendication 1 sous forme d'épimères équatoriaux.

4. [(Fluoro-4 phénoxy)-3 propyl]-1 [N-(méthoxy-4 phényl) thiocarbamoyloxy[-4 trans-décahydroquinoléine (forme axiale) et ses sels d'addition non toxiques selon la Revendication 1.

5. [(Bromo-4 phénoxy)-3 propyl]-1 [N-(méthoxy-4 phényl) thiocarbamoyloxy]-4 trans-décahydroquinoléine (forme axiale) et ses sels d'addition non toxiques selon la Revendication 1.

6. [(Méthoxy-4 phénoxy)-3 propyl]-1 [N-(méthoxy-4 phényl) thiocarbamoyloxy]-4 trans-décahydroquinoléine (forme axiale) et ses sels d'addition non toxiques selon la Revendication 1.

7. [(Diméthoxy-3,4 phénoxy)-3 propyl]-1 [N-(méthoxy-4 phényl) thiocarbamoyloxy]-4 trans-décahydroquinoléine (forme axiale) et ses sels d'addition non toxiques selon la Revendication 1.

8. [N-(Méthoxy-4 phényl) thiocarbamoyloxy]-4 [(méthylènedioxy-3,4 phénoxy)-3 propyl]-1 trans-décahydroquinoléine (forme axiale) et ses sels d'addition non toxiques selon la Revendication 1.

9. Dérivés de décahydroquinoléinol selon l'une quelconque des Revendications 1 à 8 caractérisés en ce que le sel d'addition non toxique est le chlorhydrate.

10. Composition pharmaceutique ou vétérinaire caractérisée en ce qu'elle contient, à titre de principe actif, au moins un dérivé de décahydroquinoléinol selon l'une quelconque des Revendications 1 à 9, en association avec un véhicule pharmaceutique ou un excipient approprié.

11. Procédé de préparation de dérivés de décahydroquinoléinol selon la Revendication 1, caractérisé en ce que l'on fait réagir, à température ambiante et dans un solvant organique inerte, une hydroxy-4

trans-décahydroquinoléine N-substituée de formule générale :

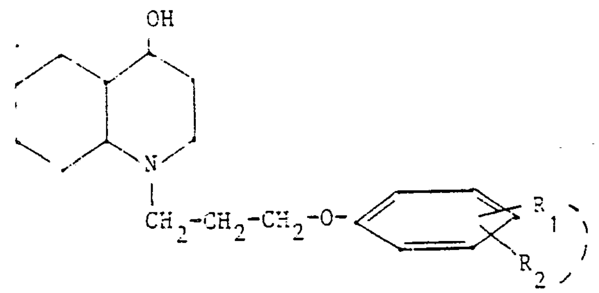

dans laquelle $R_1$ et $R_2$ ont la même signification que dans la Revendication 1, avec un composé de formule générale :

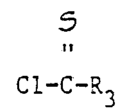

dans laquelle $R_3$ représente un atome de chlore ou un radical phénoxy, la réaction ayant lieu en présence d'une amine tertiaire lorsque $R_3$ représente un radical phénoxy pour obtenir un dérivé thiocarbonyloxy de formule générale :

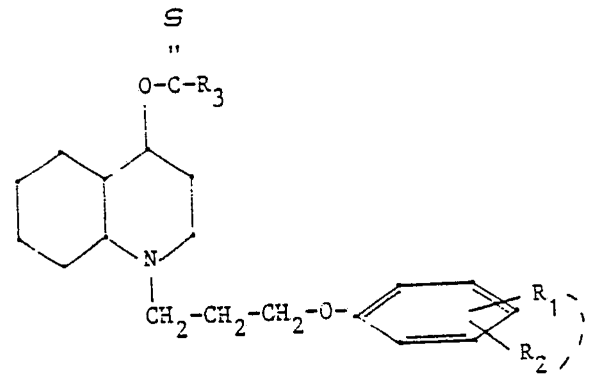

dans laquelle $R_1$, $R_2$ et $R_3$ ont la même signification que dans la Revendication 1, que l'on condense avec une amine de formule générale :

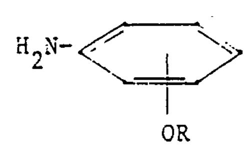

dans laquelle R a la même signification que dans la Revendication 1, dans un solvant inerte et à la température ambiante, pour former l'ester thiocarbamique de décahydroquinoléinol correspondant sous forme de sa base libre, lequel peut être traité, par la suite, si on le désire, avec un acide organique ou inorganique pour obtenir un sel d'addition non toxique.

**Claims**

1. Dérivatives of decahydroquinolinol corresponding with the general formula :

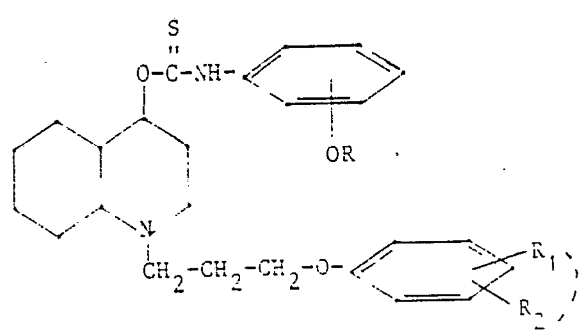

and their non-toxic addition salts, where R represents an alkyl radical having from 1 to 3 carbon atoms, $R_1$ represents fluorine, chlorine, bromine, or methoxy, $R_2$ represents hydrogen or methoxy or $R_1$ and $R_2$, when they are taken together, represent a 2,3-methylenedioxy or 3,4-methylenedioxy grouping.

2. Derivatives of decahydroquinolinol in accordance with Claim 1 in the form of axial epimers.

3. Derivatives of decahydroquinolinol in accordance with Claim 1 in the form of equatorial epimers.

4. 1-[3-(4-Fluoro-phenoxy)-propyl]-4-[N-(4-methoxy-phenyl)-thiocarbamoyloxy]-trans-decahydroquinoline (axial form) and its non-toxic addition salts in accordance with Claim 1.

5. 1-[3-(4-Bromo-phenoxy)-propyl]-4-[N-(4-methoxy-phenyl)-thiocarbamoyloxy]-trans-decahydróquinoline (axial form) and its non-toxic addition salts in accordance with Claim 1.

6. 1-[3-(4-Methoxy-phenoxy)-propyl]-4-[N-(4-methoxy-phenyl)-thiocarbamoyloxy]-trans-decahydroquinoline (axial form) and its non-toxic addition salts in accordance with Claim 1.

7. 1-[3-(3,4-Dimethoxy-phenoxy)-propyl]-4-[N-4-methoxy-phenyl)-thiocarbamoyloxy]-trans-decahydroquinoline (axial form) and its non-toxic addition salts in accordance with Claim 1.

8. 4-[N-(4-Methoxy-phenyl)-thiocarbamoyloxy]-1-[3-(3,4-methylenedioxy phenoxy)-propyl]-trans-decahydroquinoline (axial form) and its non-toxic addition salts in accordance with Claim 1.

9. Derivatives of decahydroquinolinol in accordance with any one of the Claims 1 to 8 characterized in that the non-toxic addition salt is the hydrochloride.

10. Pharmaceutical or veterinary composition characterized in that it contains, as an active constituent, at least one decahydroquinolinol derivative in accordance with one of the Claims 1 to 9, in association with an appropriate excipient or pharmaceutical vehicle.

11. Process for the preparation of decahydroquinolinol derivatives in accordance with Claim 1, characterized in that a reaction is caused at ambient temperature and in an inert organic solvent between an N-substituted 4-hydroxy trans-decahydroquinoline of general formula :

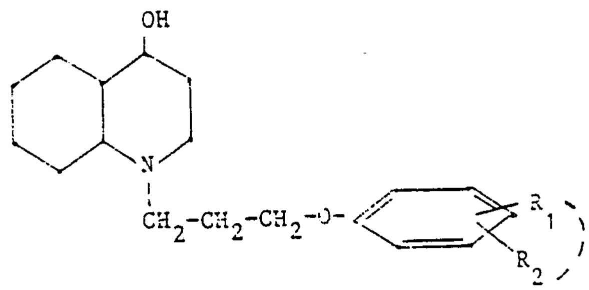

where $R_1$ and $R_2$ have the same meanings as in Claim 1, with a compound of general formula :

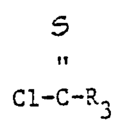

where $R_3$ represents an atom of chlorine or a phenoxy radical, a reaction taking place in the presence of a tertiary amine when $R_3$ represents a phenoxy radical to obtain a thiocarbonyloxy derivate of general formula :

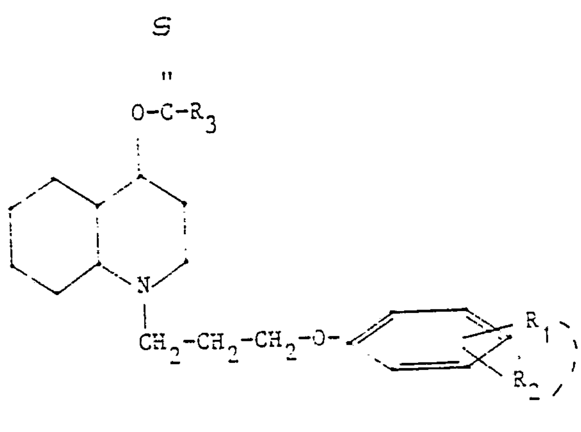

where $R_1$, $R_2$, and $R_3$ have the same meanings as in Claim 1, which is condensed with an amine of general formula :

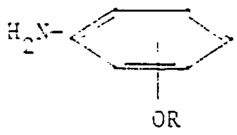

where R has the same meaning as in Claim 1, in an inert solvent and at ambient temperature, to form the thiocarbamic ester of decahydroquinolinol corresponding with the form of its free base, which ester may subsequently be treated, if so desired, with an organic or inorganic acid to obtain a non-toxic addition salt.

**Patentansprüche**

1. Decahydrochinoliderivate entsprechend der allgemeinen Formel

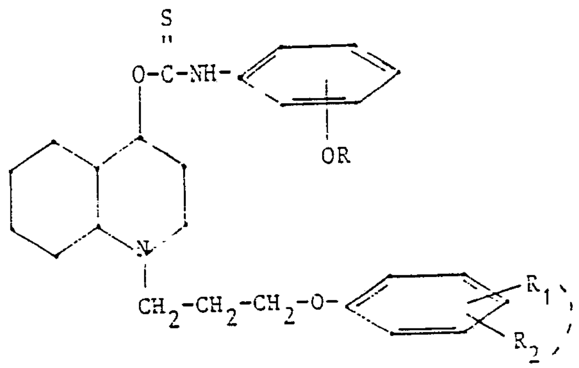

sowie ihre nicht-toxischen Additionssalze, in welchen R einen Alkylrest mit 1 bis 3 Kohlenstoffatomen bedeutet, $R_1$ Fluor, Chlor, Brom oder Methoxy bedeutet, $R_2$ Wasserstoff oder Methoxy bedeutet, oder $R_1$ und $R_2$ zusammengenommen eine 2,3-Methylen-dioxy- oder 3,4-Methylen-dioxygruppe bedeuten.

2. Decyhydrochinolinolderivate nach Anspruch 1 in der Form der axialen Epimeren.

3. Decahydrochinolinolderivate nach Anspruch 1 in der Form der äquatorialen Epimeren.

4. 1-[3-(4-Fluorphenoxy)-propyl]-4-[N-(4-methoxyphenyl) thiocarbamoyloxy]-trans-decahydrochinolin (axiale Form) und dessen nicht-toxische Additionssalze nach Anspruch 1.

5. 1-[3-(4-Bromphenoxy)-propyl]-4-[N-(4-methoxyphenyl)-thiocarbamoyloxy]-trans-decahydrochinolin (axiale Form) und dessen nicht-toxische Additionssalze nach Anspruch 1.

6. 1-[3-(4-Methoxyphenoxy)-propyl]-4-[N-(4-methoxyphenyl)-thiocarbamoyloxy]-trans-decahydrochinolin (axiale Form) und dessen nicht-toxische Additionssalze nach Anspruch 1.

7. 1-[3-(3,4-Dimethoxyphenoxy)-propyl]-4-[N-(4-methoxyphenyl)-thiocarbamoyloxy]-trans-decahydrochinolin (axiale Form) und dessen nicht-toxische Additionssalze nach Anspruch 1.

8. 4-[N-(4-Methoxyphenoxyl)-thiocarbamoyloxy]-1-[3,4-methylendioxyphenoxy)-propyl]-1-trans-decahydrochinolin (axiale Form) und dessen nicht-toxische Additionssalze nach Anspruch 1.

9. Decyhydrochinoliderivate nach irgendeinem der Ansprüche 1 bis 8, dadurch gekennzeichnet, daß das nicht-toxische Salz das Hydrochlorid ist.

10. Pharmazeutische oder veterinärmedizinische Zusammensetzung, dadurch gekennzeichnet, daß sie als Wirkstoff mindestens ein Decahydrochinolinolderivat nach irgendeinem der Ansprüche 1 bis 9 in Verbindung mit einem pharmazeutischen Medium oder einem geeigneten Träger enthält.

11. Verfahren zur Herstellung von Decahydrochinolinolderivaten nach Anspruch 1, dadurch gekennzeichnet, daß man bei Umgebungstemperatur und in einem inerten organischen Lösungsmittel ein N-substituiertes 4-Hydroxy-trans-decahydrochinolin der allgemeinen Formel

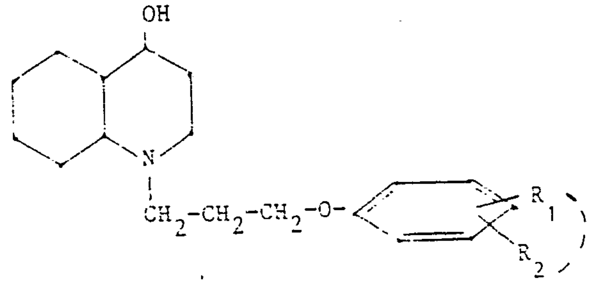

in welcher $R_1$ und $R_2$ die in Anspruch 1 genannte Bedeutung haben, mit einer Verbindung der allgemeinen Formel

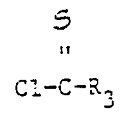

in welcher $R_3$ ein Chloratom oder einen Phenoxyrest bedeutet, reagieren läßt, wobei die Reaktion in Anwesenheit eines tertiären Amins erfolgt, wenn $R_3$ für einen Phenoxyrest steht, um ein Thiocarbonyloxyderivat der allgemeinen Formel

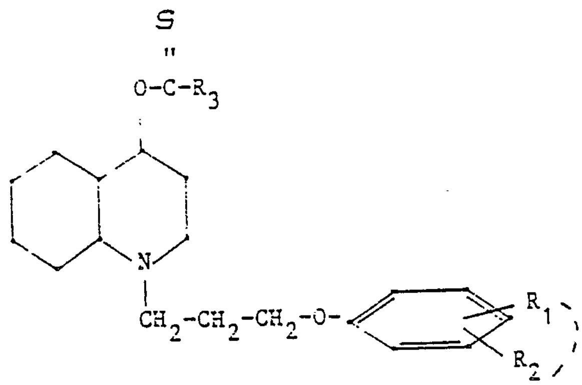

in welcher $R_1$, $R_2$ und $R_3$ dieselbe Bedeutung wie in Anspruch 1 haben, zu erhalten, das man mit einem Amin der allgemeinen Formel

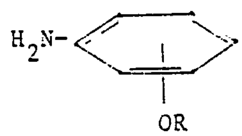

in welcher R dieselbe Bedeutung wie in Anspruch 1 hat, in einem inerten Lösungsmittel und bei Umgebungstemperatur kondensiert, um den Thiocarbaminester des entsprechenden Decahydrochinolinols in Form der freien Base zu bilden, der anschließend gegebenenfalls mit einer organischen oder anorganischen Säure behandelt wird, um ein nicht-toxisches Additionssalz zu erhalten.